# FASCICULE DE BREVET EUROPEEN

(11) **EP 0 941 098 B1**
(45) Date de publication et mention de la délivrance du brevet: **14.05.2003**
(21) Numéro de dépôt: 97939885.6
(22) Date de dépôt: 12.09.1997
(51) Int. Cl.: A61K 31/655, A61P 35/00

(54) **PROCEDE D'INHIBITION DE BIOSYNTHESE DE TRIPHOSPHATE DE DESOXYRIBONUCLEOTIDE**
VERFAHREN ZUR UNTERBRECHUNG DER DESOXYRIBONUKLEOTIDTRIPHOSPHAT-BIOSYNTHESE
METHOD FOR INHIBITING DESOXYRIBONUCLEOTIDE TRIPHOSPHATE BIOSYNTHESIS

(30) Priorité: 13.09.1996 BE 9600772
(43) Date de publication de la demande: 15.09.1999
(73) Titulaire: Previsan AG, 6301 Zug (CH)
(72) Inventeur: VANDEVELDE, Michel, B-1000 Bruxelles (BE); MARGERY, Hélène, B-1301 Bierges (BE)
(74) Mandataire: Claeys, Pierre
(86) Numéro de dépôt international: BE9700104
(87) Numéro de publication internationale: WO98010772

(56) Documents cités:
- WO-A-91/07876
- WO-A-91/16054
- R.E. MEYN ET AL.: "Post-radiation treatment of CHO cells with diamide inhibits DNA strand break rejoining." RADIAT. RES., vol. 94, no. 3, 1983, page 614 XP002032189
- J.F. WARD ET AL.: "Effects of inhibitors of DNA strand break repair on HeLa cell radiosensitivity." CANCER RES., vol. 44, no. 1, 1984, pages 59-63, XP000672939

## Description

La présente invention est relative à un procédé d'inhibition de biosynthèse de triphosohate de désoxyribonucléotide par des cellules isolées notamment animales, humaines ou végétales.

Il est connu que l'information génétique est supportée par de l'acide désoxyribonucléique (ADN) contenu dans le noyau cellulaire. L'ADN est formé d'une double hélice composée de nucléotides qui est fondamentale pour les espèces vivantes, tant animales que végétales.

Les nucléotides sont, comme on le sait, formés d'un sucre, d'une base azotée hétérocyclique et d'au moins un groupe phosphate. Dans les nucléotides formant l'ADN on trouve quatre phosphates : le triphosphate de désoxyguanidine (dGTP), le triphosphate de désoxythymidine (dTTP), le triphosphate de désoxyadénosine (dATP) et le triphosphate de désoxycytidine (dCTP).

Depuis quelques années on a observé que les cellules cancéreuses, parce qu'elles se divisent rapidement, consomment une grande quantité de triphosphates nucléotidiques. La recherche s'est portée dès lors sur des médicaments susceptibles d'inhiber la formation des désoxyribonucléotides, comme par exemple du 5-fluorouracile, de l'aminoptérine et de l'améthoptérine (voir J. DAVID RAWN, Biochemistry, p. 648). On peut aussi citer l'hydroxyurée qui inhibe de manière non sélective la ribonucléotide-réductase, et de cette façon l'ensemble des nucléotides impliqués dans la synthèse de l'ADN (voir DRUG, Facts and comparisons, J.B. LIPPINCOTT COMPANY, 1990, p. 2258 et 2259; P. REICHARD, From RNA to DNA, why so many ribonucléotide reductases ?, Science, vol. 260, 1993, p. 1773-1776).

L'inconvénient de ces produits est que, vu leur manque de sélectivité, ils inhibent des mécanismes qui sont indispensables aux cellules saines, non cancéreuses, et qui sont en rapport avec les désoxynucléotides, comme par exemple le transport d'énergies intracellulaires et les réactions enzymatiques qu'ils catalysent. Il s'ensuit une toxicité importante pour l'ensemble des cellules, lors d'un traitement par ces produits.

On a également déjà examiné l'effet d'un analogue du dCTP, la cytosine arabinoside ou cytarabine, qui agit en prenant la place de la molécule naturelle dans l'ADN cellulaire, par un phénomène de compétition (voir DRUG, op. cit., p. 2192-2196). Ce produit n'est pas actif par voie orale et doit être administré avec une grande prudence.

Des études fondamentales ont d'ailleurs été menées pour tenter de combiner l'hydroxyurée à la cytarabine. L'effet attendu était de remplacer par de la cytarabine la quantité de dCTP réduite sous l'action de l'hydroxyurée (voir abrégé fourni par la base de données MEDLINE EXPRESS de : SCHILSKY R.L. et cons., Laboratory and Clinical studies of biochemical modulation by hydroxyurea, Semin. Onc. 1992, Juin 19 (3 Suppl. 9) : 84-89).

D'autres lignées cellulaires que les lignées cancéreuses peuvent avoir un taux de prolifération exagéré. Il en est ainsi de cellules lymphocytaires et des cellules musculaires lisses des vaisseaux sanguins lors de greffes d'organes (allogreffes).

Il a déjà été tenté de réguler ces cellules en limitant leur taux de désoxynucléotides. On peut citer par exemple l'acide mycophénolique (MPA) ou un de ses dérivés qui bloque la déshydrogénase de monophosphate d'inosine, ce qui entraîne une diminution de dGTP intracellulaire et par conséquent entrave la synthèse d'ADN par ces cellules (voir PICHIMAYR R., Placebo-controlled study of mycophenolate mofetil combined with cyclosporin and corticosteroids for prevention of acute rejection, The Lancet, vol. 345, May 27, 1995, p. 1321-1325; SOLLINGER H.W., Mycophenolate mofetil for the prevention of acute rejection in primary cadaveric renal allograft recipients, Transplantation, vol. 60, 225-232, No 3, 1995; GREGORY C.R., Treatment with rapamycin and mycophenolic acid reduces arterial intimal thickening produced by mechanical injury and allows endothelial replacement, Transplantation, vol. 59, 655-661, No 5, 1995).

Il est connu enfin que des maladies virales, et en particulier le SIDA, réclament des cellules infectées, pour la réplication des virus, un matériel génétique important. On a découvert récemment que l'acide mycophénolique précité pouvait, étant donné son action d'inhibition sur la formation de dGTP dans les cellules, bloquer in vitro l'activité de la transcriptase inverse et avoir donc un effet anti-VIH (V. HIROSHI ICHIMURA et J.A. LEVY, Polymerase substrate depletion : A novel strategy for inhibiting the réplication of the human immunodeficiency virus, Virology 211, 554-560, 1995).

La présente invention a pour but de mettre au point un procédé d'inhibition de biosynthèse de triphosphates de désoxyribonucléotides par des cellules isolées animales, humaines ou végétales qui ne présente pas les inconvénients précités, en particulier une toxicité inacceptable pour des cellules saines, et qui permette ainsi d'empêcher une production cellulaire importante et anormale d'acide désoxyribonucléique, pouvant donner lieu par exemple à une prolifération cellulaire de type cancéreux.

On résout ce problème par un procédé tel que décrit au début, comprenant une inhibition de formation d'au moins un triphosphate de désoxyribonucléotide par ces cellules lors d' une application sur lesdites cellules d'au moins un des dérivés azoïques répondant à la formule 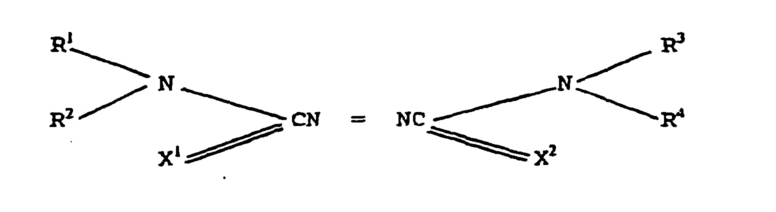 dans laquelle R¹, R², R³ et R⁴ sont identiques ou différents et représentent chacun un atome d'hydrogène ou d'halogène ou un radical d'hydrocarbure aliphatique ou aromatique, éventuellement substitué, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs isomères.

Suivant une forme de réalisation de l'invention, dans la formule ci-dessus, R₁ à R₅ représentent chacun un radical d'hydrocarbure aliphatique ou aromatique comportant de 1 à 6 atomes de carbone. Par exemple, le dérivé azoïque est choisi parmi le groupe comprenant des dérivés d'azobisformamidine, tels que de la 1,1'-azobisformamidine, de la 1,1-azobisnitroformamidine, de la 2,2'-azobisméthylformamidine, de la 1,1'-azobisfluoroformamidine, de la 1-monochloro-azobisformamidine, et de la azobis-[chloroformamidine]; des dérivés d'azobisformamide, tels que du 1,1'-azobisformamide et du diméthylazobisformamide, et de la 1,1'-(azodicarbonyl)-dipipéridine.

Plusieurs de ces dérivés azoïques sont des composés connus, en particulier pour leur activité antivirale, notamment contre les virus du groupe rétrovirus, en particulier le virus du SIDA (voir EP-A-0504184 et EP-A-0524961).

La préparation de la 1,1'-azobisformamidine et du 1,1'-azobisformamide a déjà été réalisée à la fin du siècle passé par J. THIELE (v. The Merck Index, 10 éd. 919, Rahway, 1983; F.C. SCHMELKES et cons., N,N'-Dichloroazodicarbonamidine (azochloramid), a N-chloro derivative of the oxidant in an oxidation-reduction system, Journal of American Chemical Society, 56, 1610, 1934; FR-B-2056874; US-A-3225026; US-A-3684713). Le 1,1'-azobisformamide est connu comme adjuvant dans la farine alimentaire (US-A-2903361). Le 1,1'-azobisdiméthylformamide est également connu depuis longtemps par son action oxydante intracellulaire sur le glutathion de cellules du sang humain (N.S. KOSOWER et cons., Diamide, a new reagent for the intracellular oxidation of glutathione to the disulfide, Biochemical and Biophysical Research Communications, vol. 37, n° 4, 1969) ainsi que par son initiation d'un efflux de Ca²⁺ supplémentaire depuis le foie de rats perfusé (H. SIES et cons., Hepatic calcium efflux during cytochrome P-450-dependent drug oxidations at the endoplasmic reticulum in intact liver, Proc. Natl. Acad. Sci. USA, Vol. 78, n° 6, p. 3358-3362). Cette substance a aussi été étudiée pour son inhibition de la réparation de petites ruptures de brins d'ADN causées par irradiation de cellules en milieu hypoxique à l'aide de rayonnements ionisants (R.E. MEYN et cons., Post-radiation treatment of CHO cells..., Radiation Research, vol. 94, n° 3, 1983, p. 614; J.F. WARD et cons., Effects of Inhibitors of DNA Strand Break Repair..., Cancer Research, 44, 1984, p. 59-63). On connaît également depuis longtemps la 1,1'-azobisnitroformamidine (W.D. KUMLER, The Dipole Moments, Ultraviolet spectra and structure of azo-bis-(chloroformamidine) and azo-bis-(nitroformamidine), Journal of American Chemical Society, 75, 3092, 1953). La chloroazodine utilisée suivant l'invention est également connue depuis longtemps comme désinfectant (voir US-A-2073256 et GB-A-421006).

L'observation de l'effet de ces substances sur des cellules in vitro, puis dans des essais cliniques n'avait pas permis de comprendre leur mode d'action sur les virus VIH. Divers examens ont été entrepris dans ce but. Ils ont permis de conclure que le 1,1'-azobisformamide (ADA) n'inhibe pas la transcriptase inverse dans un système d'essai exempt de cellules, à des concentrations CI₉₀. Un traitement simultané de cellules MT₄ avec cette substance et VIH-1 n'interfère pas sur l'intégration d'ADN proviral. De plus, l'ADA est apparu comme n'inhibant pas une transactivation Tat d'une expression de gène induit par LTR de VIH-1 (M. VANDEVELDE et cons., ADA, a potential anti-HIV drug, Aids research and human retroviruses, vol. 12, n° 7, 1996, p. 567-568).

Il résulte de ces tests que l'ADA n'agit pas comme les inhibiteurs de transcriptase inverse connus et habituellement utilisés dans le traitement du SIDA, tels que l'AZT, le ddI, le ddC et d'autres. Son action se situe à un stade posttranscriptionnel non identifié. Il n'agit pas non plus comme la 7-chloro-5-(2-pyrrile)-3H-1,4-benzodiazépin-2(H)-one récemment développée par la firme ROCHE.

Enfin, des essais encore plus récents sur l'ADA ont montré qu'au stade posttranscriptionnel l'ADA n'avait pas d'effet inhibiteur de la protéase et qu'il n'agissait donc pas comme les inhibiteurs de protéase récemment appliqués dans le traitement du SIDA.

Malgré ces échecs successifs dans la compréhension du mode d'action de l'ADA sur les virus VIH, un nouvel essai a été mis en oeuvre pour déterminer si cette substance n'agissait pas sur les cellules traitées de la même manière que l'hydroxyurée. Cette dernière substance est formée d'une molécule apparentée de loin à l'ADA, bien qu'il ne s'agisse pas d'un dérivé azoïque.

Cet essai comparatif est décrit de manière plus détaillée dans les exemples qui suivent.

Il résulte de cet essai que, d'une part, l'ADA agit bien sur la biosynthèse des triphosphates de désoxyribonucléotides cellulaires, ce qui permet d'expliquer son mode d'action posttranscriptionnel sur les virus VIH, mais que, d'autre part, son action est d'une tout autre nature que celle de l'hydroxyurée L'ADA n'est en effet pas un inhibiteur de la ribonucléotide réductase non sélectif, mais au contraire un dérivé inhibant de manière primordiale la formation du dCTP contenu dans les cellules. Or il faut noter que les dCTP forment le groupe de phosphates désoxynucléotidiques dont la concentration dans les cellules est la plus faible. Une réduction ou une disparition sélective de ces dCTP dans la cellule empêche donc celle-ci de réaliser une biosynthèse d'ADN, en nécessitant pour cela une concentration en substance active nettement moindre que celle nécessaire pour inhiber l'ensemble des désoxynucléotides, comme le fait l'hydroxyurée.

L'inhibition de la biosynthèse, qui se produit dans le cas de l'ADA par une inhibition préférentielle de la formation de triphosphates de désoxycytidine, pourrait avoir lieu à la suite d'une action de l'ADA sur différentes cibles enzymatiques des cellules. On pourrait prévoir que l'ADA ait un effet inhibiteur sur l'UMP/CMP kinase (kinase de monophosphate d'uracile/monophosphate de cytidine) ce qui bloquerait la formation de diphosphate de désoxycytidine (dCDP) à partir de monophosphate de désoxycytidine (dCMP), ainsi que la formation de diphosphate de cytidine (CDP) à partir de monophosphate de cytidine (CMP). Un effet de l'ADA sur la synthétase de triphosphate de cytidine (CTP), qui bloquerait la formation de cette substance à partir de triphosphate d'uracile (UTP), pourrait aussi être envisagé. D'autres cibles pourraient aussi être envisagées, bien que moins probables dans le cas spécifique de l'ADA, comme par exemple la NDP kinase entre autres.

De plus, il faut noter que l'absence de toxicité de l'ADA pour l'être humain est depuis longtemps connue (voir B.L. OSER et cons., Studies of the Safety of azodicarbonamide as a flour-maturing agent, Toxicology and applied Pharmacology 7, 445-472, 1965).

Par ailleurs, un essai a déjà été effectué sur des volontaires sains. Pendant 30 jours, ils ont été traités par 1500 mg par jour d'ADA, en trois prises de 500 mg, et cela sans effet secondaire (voir EP-0524961, exemple 12). Des essais cliniques ont ensuite été entrepris. Dix volontaires ont pris de l'ADA durant 3 mois, aux doses de trois fois 1 g/jour le premier mois, trois fois 2 g/jour le deuxième mois et trois fois 3 g/jour le troisième mois. Aucun effet secondaire sérieux n'a été observé, si ce n'est un épisode de lithiase rénale à la dose de 9 g/jour chez l'un des patients, à la suite d'une accumulation du catabolite du produit (la biurée) dans les reins.

Il est également connu que le 1,1'-azobisdiméthylformamide dans le traitement de cellules saines ne présente pas de toxicité vis-à-vis de celles-ci même à des concentrations relativement élevées (voir EP-A-0524961, exemples 9, a) et 10, a)). Ce même effet a été constaté pour la 1,1'-azobisdiméthylformamidine (voir EP-A-0524961, exemple 8, a) et pour le 1,1'-azobisformamide (voir EP-A-0524961, exemple 11, a)).

Enfin, il est également connu que la chloroazodine à des concentrations de 660 µg/ml ne diminue pas la viabilité de cellules saines et n'atteint pas le seuil de toxicité pour l'environnement, par évaluation de la toxicité aiguë sur le poisson, (voir EP-A-0504184, exemples 6 et 12).

Il en résulte que les dérivés à utiliser suivant l'invention non seulement permettent d'agir sur la biosynthèse de triphosphates de désoxyribonucléotides dans les cellules traitées, à des dosages relativement faibles, mais offrent en outre une toxicité propre extrêmement faible en soi vis-à-vis du corps humain ou des cellules saines traitées.

Suivant une forme de réalisation de l'invention, l'application d'au moins un des dérivés azoïques indiqués est effectuée sur des cellules isolées de macroorganismes ou des cellules de microorganismes, qui par exemple proviennent de cultures cellulaires. On peut envisager aussi suivant l'invention une telle application sur des cellules d'un organisme ou tissu multicellulaire extrait d'un corps humain ou animal, par exemple sur des cellules de vaisseaux sanguins ou des cellules d'un échantillon de sang ou de lymphe, ainsi que sur des cellules d'une greffe destinée à être introduite, après l'application, dans un corps humain ou animal, par exemple un coeur ou un rein. Par greffe, on peut entendre une allogreffe à introduire dans un autre corps humain ou animal et par organisme ou tissu multicellulaire extrait on peut entendre que l'extraction est définitive. On peut aussi envisager, par ces dérivés azoïques, un traitement phytosanitaire de végétaux, par exemple de semences ou de plantes développées.

D'autres détails concernant le procédé d'inhibition suivant l'invention sont indiqués dans les revendications 1 à 11.

L'invention concerne également une utilisation d'au moins un dérivé azoïque, tel qu'indiqué précédement, pour la fabrication de médicaments à mettre en oeuvre dans le traitement ou la prophylaxie d'affections humaines ou animales donnant lieu à une production cellulaire importante et anormale d'acide désoxyribonucléique, à l'exception des maladies virales, notamment des infections par les virus du groupe rétrovirus. On peut prévoir notamment la fabrication de médicaments anticancéreux, tels que des médicaments contre les tumeurs liquides et solides, comme les antileucémiques et les antitumoraux.

D'autres détails concernant l'utilisation suivant l'invention de dérivés azoïques indiqués sont donnés en particulier dans les revendications 12 et 13.

L'invention va à présent être expliquée de manière plus détaillée à l'aide d'exemples décrits ci-dessous.

### Exemple 1

Essai comparatif entre l'ADA et l'hydroxyurée.

Cet essai a été effectué par l'Antiviral Research Laboratory du Center for Drug Evaluation and Research de la Food and Drug Administration des Etats-Unis d'Amérique.

### Méthode :

### Cellules.

Des cellules humaines A3,01 ont été incubées avec des concentrations 0, 10, 100, 200 micromolaires d'ADA, et avec une concentration 100 micromolaire d'hydroxyurée dans du RPMI 1640 additionné de 10% de sérum foetal de veau, 4mM de L-glutamine, dans une atmosphère humidifiée composée de 95% d'air et de 5% de CO₂.

Des cellules en croissance logarithmique ont été utilisées pour des déterminations de triphosphates de désoxynucléotides (dNTP) et de triphosphates de ribonucléotides (rNTP).

Préparation des extraits cellulaires pour HPLC.

Après incubation, des extraits méthanoliques à 60% ont été préparés à partir de cellules A3,01, inactivés à la chaleur, et analysés comme décrit dans Ford, H. Jr et al, Cancer Research, 51 : 3733-3740, 1991.

Détermination des pools intracellulaires de rNTP et dNTP par gradient d'échange d'ions HPLC.

Les ribonucléotides cellulaires ont été mesurés par HPLC échangeuse d'ions sur des colonnes Partisil 10 Sax, comme décrit dans Ford et al., op. cit.

Les désoxynucléotides ont été déterminés en utilisant du periodate de sodium pour éliminer les ribonucléotides; les désoxyribonucléotides ont alors été déterminés par HPLC échangeuse d'ions comme décrit dans Hao Z. et al, Mol. Pharmacol., 34, 431-435, 1988.

### Résultats.

Effet de l'ADA sur les pools de rNTP dans les cellules A3,01

| | UTP* (nmole/10⁴ cellules) | CTP* | ATP* | GTP* |
|---|---|---|---|---|
| ADA (10⁻⁶ M) | | | | |
| 0 | 0,71 | 0,24 | 2,51 | 0,38 |
| 10 | 0,87 | 0,28 | 2,85 | 0,45 |
| 100 | 1,10 | 0,31 | 2,64 | 0,47 |
| 200 | 0,49 | 0,09 | 1,31 | 0,22 |
| Hydroxyurée (10⁻⁶ M) | | | | |
| 100 | 1,01 | 0,34 | 3,69 | 0,93 |

| | | | | |
|---|---|---|---|---|
| * UTP = triphosphate d'uracile; CTP = triphosphate de cytosine; ATP = triphosphate d'adénosine; GTP = triphosphate de guanosine. | | | | |

Effet de l'ADA sur les pools de dNTP dans les cellules A3,01

| | dTTP (pmole/10⁶ cellules) | dCTP | dATP | dGTP |
|---|---|---|---|---|
| ADA (10⁻⁶ M) | | | | |
| 0 | 39,7 | 12,7 | 50,3 | 17,2 |
| 10 | 39,2 | 6,5 | 53,6 | 19,4 |
| 100 | 40,3 | 6,2 | 52,1 | 17,1 |
| 200 | 19,1 | 2,9 | 22,8 | 15,1 |
| Hydroxyurée (10⁻⁶ M) | | | | |
| 100 | 21,6 | 3,6 | 9,0 | 19,3 |

Le composé azodicarbonamide montre donc un effet inhibiteur préférentiel sur le pool de dCTP (50% d'inhibition à des concentrations de 10 µM et de 100 µM).

A des concentrations plus hautes, l'ADA montre un effet plus cyototoxique, qui résulte d'une diminution des pools de rNTP et des pools de dNTP.

L'hydroxyurée, un inhibiteur connu de la ribonucléotide réductase, est très différent de l'ADA. Il diminue les pools de dATP, dCTP et dTTP alors qu'il augmente les pools de rNTP à la concentration de 100 microM. Le rapport entre rNTP/dNTP dans les cellules traitées à l'hydroxyurée (68,2) (diminution des pools de dNTP et augmentation des pools de rNTP) par rapport à celui observé dans les cellules non traitées (24,33) indique que l'hydroxyuré est un inhibiteur spécifique de la ribonucléotide réductase alors que l'effet de l'ADA est préférentiellement spécifique du rapport CTP/dCTP.

### Exemple 2

L'effet préférentiel de l'ADA sur les pools de dCTP intracellulaire observé dans l'exemple 1 a été confirmé par d'autres examens effectués par l'Antiviral Research Laboratory du Center for Drug Evaluation and Research de la Food and Drug Administration des Etats-unis d'Amérique.

### Méthode :

Des cellules de sang périphérique humain provenant de donneurs sains (peripheral blood monocytic cells = PBMC) sont stimulées par de la phytohémoagglutinine (PHA) puis elles sont incubées avec des concentrations de 0, 50, 100, 200 µmoles d'ADA dans un milieu de culture approprié. Les extraits cellulaires ont été examinés par 3 méthodes différentes.
1°) Chromatographie en phase liquide à haute performance de l'extrait total (HPLC).
2°) Après destruction des précurseurs (uridine triphosphate, cytidine triphosphate, adénosinetriphosphate et guanidinetriphosphate) par du periodate, les extraits sont soumis à une analyse par HPLC.
3°) Analyse par une méthode F.PODER, consistant en un dosage par détection à l'aide de sondes d'ADN spécifiques et amplification.

Les résultats sont repris dans le tableau 1 ci-dessous.

**Tableau 1**

| Effet préférentiel de l'ADA sur les pools de dCTP dans des PBMC stimulées au PHA | | | | | |
|---|---|---|---|---|---|
| Méthode | HPLC | Periodate | FPODER | Moyenne | Inhibition % |
| Quantités d'ADA (µM) | dCTP | dCTP | dCTP | dCTP | |
| 0 | 3,01 | 1,42 | 1,76 | 2,06 | 0 |
| 50 | 0,09 | 1,16 | 0,88 | 0,71 | 66 |
| 100 | 0,07 | 0,76 | 0,61 | 0,48 | 77 |
| 200 | 0,05 | 0,6 | 0,46 | 0,37 | 82 |

### Exemple 3

Examen de cellules Supt-1 (lignée continue de cellules lymphoblastiques humaines) qui ont été stimulées par PHA et traitées ensuite par des doses de 10 µg et de 20 µg d'ADA/ml de milieu de culture.

Le nombre de cellules a été standardisé à 5.10⁴ cellules par ml au jour 0. L'inhibition de la prolifération est exprimée en pourcentage de la prolifération du témoin.

Trois méthodes d'évaluation ont été appliquées au Laboratoire de Chimie biologique et de la Nutrition de l'Université Libre de Bruxelles ainsi qu'au Laboratoire d'immunologie de cette Université.
- L'incorporation de thymidine tritiée dans les cellules comme marqueur radiographique (expériences répétées 8 fois).
- Marquage colorimétrique de la mort cellulaire par du MTT (expériences répétées 12 fois).
- Méthode d'exclusion au bleu de trypan qui est un témoin de l'intégrité des membranes cellulaires (expériences répétées 8 fois).

Les résultats sont repris dans le tableau 2 ci-dessous. Il en ressort un effet net sur la prolifération d'une lignée continue de cellules humaines.

**Tableau 2**

| Inhibition de la prolifération de cellules SUP-T1 (examen après 48 heures) | | |
|---|---|---|
| Méthode | ADA 20 µg/ml | ADA 10 µg/ml |
| 3H=Thymidine 18 h | 39 % ± 18 | 15 % ± 6 |
| MTT | 43 % ± 8 | 19 % ± 10 |
| Bleu de trypan | 37 % ± 17 | 24 % ± 10 |

### Exemple 4

Gelules à administrer par voie orale.

Composition d'une gelule :
500 mg d'ADA
10 mg de monostéarate de glycérine
10 mg de dioxyde de silicium précipité
5 mg de stéarate de magnésium.

On introduit d'une manière courante cette composition dans des capsules de gélatine. On peut par exemple prévoir une administration de 2 gelules 3 fois par jour à des patients qui présentent un phénomène de prolifération cellulaire pathologique. Les gelules seront administrées seules ou en association avec tout autre traitement antiprolifératif approprié.

### Exemple 5

Tablettes enrobées.

On réalise d'une manière courante des tablettes contenant 250 mg d'azobisformamidine à l'aide des excipients suivants : hydroxypropylméthylcellulose, hydroxypropylcellulose, dioxyde de titane, polyéthylèneglycol 400, oxyde noir de fer.

Ces tablettes peuvent être administrées pour réduire de manière adéquate les dCTP intralymphocytaires (dosage par HPC après isolation des lymphocytes).

Ce traitement sera instauré afin d'obtenir une induction et le maintien d'une rémission dans la leucémie lymphatique aiguë (leucémie lymphoblastique ou lymphocytique) ou dans la leucémie myéloblastique. La substance active est administrée seule ou de préférence en association avec de la mercaptopurine ou de l'azathioprine ou toute autre drogue appropriée.

### Exemple 6

Forme à injecter.

On réalise une forme injectable à base de 1 g de diméthylazobisformamide et d'eau distillée apyrogène additionnée de NaCl.

Ces formes sont à administrer par exemple dans des phénomènes d'adénosarcome, en particulier généralisé.

Ce traitement peut être associé à de l'azathioprine, du 5-fluorouracile ou à d'autres traitements conventionnels.

### Exemple 7

Crème ou onguent.

Une crème ou un onguent est réalisé avec de l'azobis-[chloroformamidine] (chloroazodine) et comme excipient, notamment de la glycérine, de l'huile de paraffine, de la vaseline.

Cette crème peut être appliquée localement à titre de traitement adjuvant de cancers cutanés, par exemple de l'épithélioma épidermoïde.

### Exemple 8

On peut aussi prévoir des systèmes de distribution transdermique de diméthylazobisformamide.

Ces systèmes peuvent être appliqués de manière à obtenir une diminution soutenue des dCTP intracellulaires chez des patients souffrant de lymphome, hodgkinien ou non.

Ce traitement peut être combiné avec l'usage de cytarabine en injection.

## Revendications

1. Procédé d'inhibition de biosynthèse de triphosphate de désoxyribonucléotide par des cellules isolées, notamment animales, humaines ou végétales, comprenant une inhibition de formation d'au moins un triphosphate de désoxyribonucléotide par ces cellules lors d' une application sur ces cellules d'au moins un des dérivés azoïques répondant à la formule 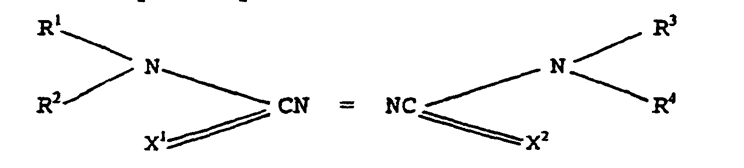 dans laquelle R¹, R², R³ et R⁴ sont identiques ou différents et représentent chacun un atome d'hydrogène ou d'halogène ou un radical d'hydrocarbure aliphatique ou aromatique, éventuellement substitué, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs isomères.

2. Procédé suivant la revendication 1, **caractérisé par** une inhibition sélective de la formation de triphosphate de désoxycytidine au cours de ladite biosynthèse.

3. Procédé suivant l'une des revendications 1 et 2, **caractérisé en ce que** R₁ à R₅ représentent chacun un radical d'hydrocarbure aliphatique ou aromatique comportant de 1 à 6 atomes de carbone.

4. Procédé suivant l'une quelconque des revendications 1 à 3, **caractérisé en ce que** le dérivé azoïque est choisi parmi le groupe comprenant des dérivés d'azobisformamidine, tels que de la 1,1'-azobisformamidine, de la 1,1'-azobisnitroformamidine, de la 2,2'-azobisméthylformamidine, de la 1,l'-azobisfluoroformamidine, de la 1-monochloro-azobisformamidine, et de la azobis-[chloroformamidine], des dérivés d'azobisformamide, tels que du 1,1'-azobisformamide et du diméthylazobisformamide, et de la 1,1'-(azodicarbonyl)-dipipéridine.

5. Procédé suivant l'une quelconque des revendications 1 à 4, **caractérisé en ce qu'**il comprend une application du dérivé azoïque sur les cellules à une concentration micromolaire de 10 à 200.

6. Procédé suivant la revendication 5, **caractérisé en ce qu'**il comprend une application du dérivé azoïque sur les cellules à une concentration micromolaire de 10 à 100.

7. Procédé suivant l'une quelconque des revendications 1 à 6, **caractérisé en ce qu'**il comprend l'application d'une composition comportant au moins un desdits dérivés azoïques et un excipient.

8. Procédé suivant l'une quelconque des revendications 1 à 7, **caractérisé en ce qu'**il comprend ladite application sur des cellules isolées de macroorganismes ou des cellules de microorganismes.

9. Procédé suivant l'une quelconque des revendications 1 à 7, **caractérisé en ce qu'**il comprend ladite application sur des cellules d'un organisme ou tissu multicellulaire extrait d'un corps humain ou animal.

10. Procédé suivant la revendication 9, **caractérisé en ce que** l'organisme ou tissu multicellulaire est une greffe.

11. Procédé suivant l'une quelconque des revendications 1 à 7, **caractérisé en ce que** ladite application est mise en oeuvre pour le traitement phytosanitaire de végétaux.

12. Utilisation d'au moins un des dérivés azoïques répondant à la formule 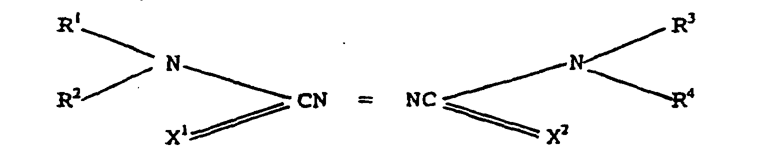 dans laquelle R¹, R², R³ et R⁴ sont identiques ou différents et représentent chacun un atome d'hydrogène ou d'halogène ou un radical d'hydrocarbure aliphatique ou aromatique, éventuellement substitué, R¹ et R² pouvant être liés ensemble pour former un noyau hétérocyclique avec leur atome d'azote adjacent, et R³ et R⁴ pouvant être liés ensemble pour former un noyau hétérocyclique avec leur atome d'azote adjacent, X¹ et X² sont identiques ou différents et représentent chacun un atome d'oxygène ou un groupe NR⁵, dans lequel R⁵ est un atome d'hydrogène ou d'halogène, un radical d'hydrocarbure aliphatique ou aromatique, éventuellement substitué, ou un groupe nitro, et dans lequel, lorsque deux groupes NR⁵ sont simultanément présents, chaque R⁵ peut être identique à ou différent de l'autre, ainsi que de leurs isomères, pour la fabrication de médicaments à mettre en oeuvre dans le traitement ou la prophylaxie d'affections humaines ou animales donnant lieu à une production cellulaire importante et anormale d'acide désoxyribonucléique, de type cancéreux, à l'exception des maladies virales, notamment des infections par les virus du groupe rétrovirus.

13. Utilisation suivant la revendication 12, pour la fabrication de médicaments anticancéreux, tels que des médicaments antileucémiques et des médicaments antitumoraux.

## Claims

1. Process for inhibiting deoxyribonucleotide triphosphate biosynthesis by isolated cells, in particular animal, human or plant cells, comprising inhibition of the formation of a least one deoxyribonucleotide triphosphate by said cells by application onto said cells of at least one of the azo derivatives of the formula 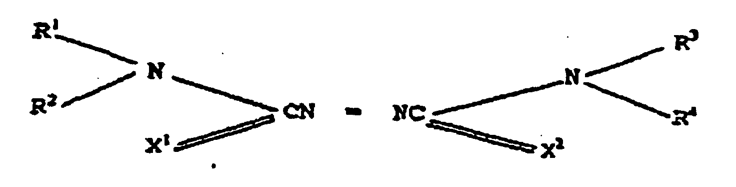 in which R¹, R², R³ and R⁴ are identical or different and each represent a hydrogen or halogen atom or an optionally substituted aliphatic or aromatic hydrocarbon residue, R¹ and R² possibly being connected together to form a heterocyclic nucleus with the nitrogen atom adjacent thereto, and R³ and R⁴ possibly being connected together to form a heterocyclic nucleus with the nitrogen atom adjacent thereto, X¹ and X² are identical or different and each represent an oxygen atom or a group NR⁵, in which R⁵ is a hydrogen or halogen atom, an optionally substituted aliphatic or aromatic hydrocarbon residue, or a nitro group, and in which, when two groups NR⁵ are simultaneously present, each R⁵ may be identical to or different from the other, as well as the isomers thereof.

2. Process according to claim 1, **characterized by** a selective inhibition of the formation of deoxycytidine triphosphate during said biosynthesis.

3. Process according to one of claims 1 and 2, **characterized in that** R¹ to R⁵ each represent an aliphatic or aromatic hydrocarbon residue comprising from 1 to 6 carbon atoms.

4. Process according to any of claims 1 to 3, **characterized in that** the azo derivative is selected from among the group comprising derivatives of azobisformamidine, such as 1,1'-azobisformamidine, 1,1'-azobisnitroformamidine, 2,2'-azobismethylformamidine, 1,1'-azobisfluoroformamidine, 1-monochloroazobisformamidine and azobis[chloroformamidine], derivatives of azobisformamide, such as 1,1'-azobisformamide and dimethylazobisformamide, and of 1,1'-(azodicarbonyl)-dipiperidine.

5. Process according to any of claims 1 to 4, **characterized in that** it comprises application of the azo derivative onto cells at a 10 to 200 micromolar concentration.

6. Process according to claim 5, **characterized in that** it comprises application of the azo derivative onto cells at a 10 to 100 micromolar concentration.

7. Process according to any of claims 1 to 6, **characterized in that** it comprises application of a composition comprising at least one of the said azo derivatives and an appropriate excipient.

8. Process according to any of claims 1 to 7, **characterized in that** it comprises said application onto cells isolated from macroorganisms or onto cells of microorganisms.

9. Process according to any of claims 1 to 7, **characterized in that** it comprises said application onto cells of an organism or multicellular tissue extracted from a human or animal body.

10. Process according to claim 9, **characterized in that** the organism or multicellular tissue is a graft.

11. Process according to any of claims 1 to 7, **characterized in that** said application is used for the phytosanitary treatment of plants.

12. Use of at least one of the azo derivatives of the formula 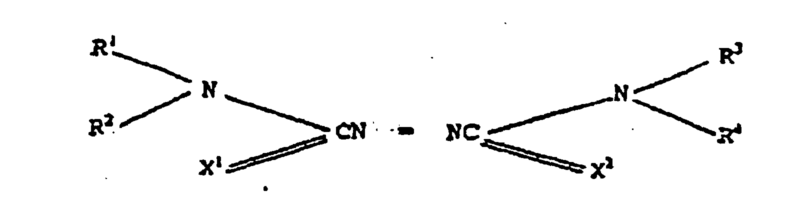 in which R¹, R², R³ and R⁴ are identical or different and each represent a hydrogen or halogen atom or an optionally substituted aliphatic or aromatic hydrocarbon residue, R¹ and R² possibly being connected together to form a heterocyclic nucleus with the nitrogen atom adjacent thereto, and R³ and R⁴ possibly being connected together to form a heterocyclic nucleus with the nitrogen atom adjacent thereto, X¹ and X² are identical or different and each represent an oxygen atom or a group NR⁵, in which R⁵ is a hydrogen or halogen atom, an optionally substituted aliphatic or aromatic hydrocarbon residue, or a nitro group, and in which, when two groups NR⁵ are simultaneously present, each R⁵ may be identical to or different from the other, as well as the isomers thereof, for the production of medicines for use in the treatment or prevention of human or animal conditions which give rise to large-scale and abnormal cellular production of deoxyribonucleic acid, of a cancerous type, with the exception of viral diseases, in particular infections by viruses of the retrovirus group.

13. Use according to claim 12 for the production of antineoplastic medicines, such as antileukemic and antitumor medicines.

## Patentansprüche

**1.** Verfahren zur Inhibierung der Desoxyribonukleotidtriphosphat-Biosynthese in isolierten Zellen, insbesondere Tier-, Human- oder Pflanzenzellen, umfassend eine Inhibierung der Bildung mindestens eines Desoxyribonukleotidtriphosphats in diesen Zellen bei Applikation mindestens eines der Azoderivate der Formel 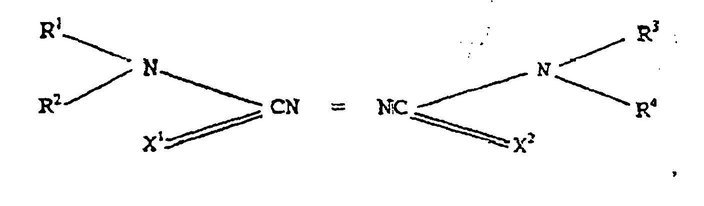 auf diese Zellen,
wobei R¹, R², R³ und R⁴ gleich oder unterschiedlich sind und jeweils ein Wasserstoffoder Halogenatom oder einen aliphatischen oder aromatischen Kohlenwasserstoffrest darstellen, der gegebenenfalls substituiert sein kann, R¹ und R² miteinander verbunden sein können, um mit dem ihnen benachbarten Stickstoffatom einen heterozyklischen Ring zu bilden, und R³ und R⁴ miteinander verbunden sein können, um mit dem ihnen benachbarten Stickstoffatom einen heterozyklischen Ring zu bilden, X¹ und X² gleich oder unterschiedlich sind und jeweils ein Sauerstoffalom oder einen Rest NR⁵ darstellen, in dem R⁵ ein Wasserstoff- oder Halogenatom, ein aliphatischer oder aromatischer Kohlenwasserstoffrest, der gegebenenfalls substituiert sein kann, oder eine Nitrogruppe ist, und in dem, falls gleichzeitig zwei Reste NR⁵ vorhanden sind, jeder Rest R⁵ gleich sein oder sich vom anderen unterscheiden kann, wie auch ihrer Isomere.

**2.** Verfahren nach Anspruch 1, **gekennzeichnet durch** eine selektive Inhibierung der Bildung von Desoxycytidintriphosphat während der Biosynthese.

**3.** Verfahren nach einem der Ansprüche 1 oder 2, **dadurch gekennzeichnet, dass** die Reste R¹ bis R⁵ jeweils einen aliphatischen oder aromatischen Kohlenwasserstoffrest mit 1 bis 6 Kohlenstoffatomen darstellen.

**4.** Verfahren nach einem der Ansprüche 1 bis 3, **dadurch gekennzeichnet, dass** das Azoderivat ausgewählt ist aus Derivaten des Azobisformamidins, wie
1,1'-Azobisformamidin, 1,1'-Azobisnitroformamidin, 2,2'-Azobismethylformamidin, 1,1'-Azobisfluorformamidin, 1-Monochlor-azobisformamidin und Azobis[chlorformamidin], Derivaten des Azobisformamids, wie 1,1'-Azobisformamid und Dimethylazobisformamid, und 1,1'-(Azodicarbonyl)dipiperidin.

**5.** Verfahren nach einem der Ansprüche 1 bis 4, **dadurch gekennzeichnet, dass** es eine Applikation des Azoderivats auf die Zellen in einer mikromolaren Konzentration von 10 bis 200 umfasst.

**6.** Verfahren nach Anspruch 5, **dadurch gekennzeichnet, dass** es eine Applikation des Azoderivats auf die Zellen in einer mikromolaren Konzentration von 10 bis 100 umfasst.

**7.** Verfahren nach einem der Ansprüche 1 bis 6, **dadurch gekennzeichnet, dass** es die Applikation einer Zusammensetzung umfasst, die mindestens eines der Azoderivate und einen Exzipienten enthält.

**8.** Verfahren nach einem der Ansprüche 1 bis 7, **dadurch gekennzeichnet, dass** es die Applikation auf isolierte Zellen von Makroorganismen oder Zellen von Mikroorganismen umfasst.

**9.** Verfahren nach einem der Ansprüche 1 bis 7, **dadurch gekennzeichnet, dass** es die Applikation auf Zellen eines Organismus oder eines mehrzelligen Gewebes, das aus einem menschlichen oder tierischen Körper entnommen wurde, umfasst.

**10.** Verfahren nach Anspruch 9, **dadurch gekennzeichnet, dass** der mehrzellige Organismus oder das mehrzellige Gewebe ein Transplantat ist.

**11.** Verfahren nach einem der Ansprüche 1 bis 7, **dadurch gekennzeichnet, dass** die Applikation zur phytosanitären Behandlung von Pflanzen verwendet wird.

**12.** Verwendung mindestens eines der Azoderivate der Formel 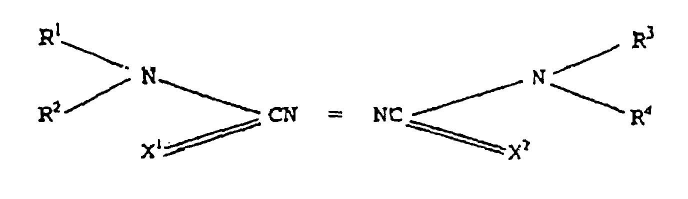 wobei R¹, R², R³ und R⁴ gleich oder unterschiedlich sind und jeweils ein Wasserstoffoder Halogenatom oder einen aliphatischen oder aromatischen Kohlenwasserstoffrest darstellen, der gegebenenfalls substituiert sein kann, R¹ und R² miteinander verbunden sein können, um mit dem ihnen benachbarten Stickstoffatom einen heterozyklischen Ring zu bilden, und R³ und R⁴ miteinander verbunden sein können, um mit dem ihnen benachbarten Stickstoffatom einen heterozyklischen Ring zu bilden, X¹ und X² gleich oder verschieden sind und jeweils ein Sauerstoffatom oder einen Rest NR⁵ darstellen, in dem R⁵ ein Wasserstoff- oder Halogenatom, ein aliphatischer oder aromatischer Kohlenwasserstoffrest, der gegebenenfalls substituiert sein kann, oder eine Nitrogruppe ist, und in dem, falls gleichzeitig zwei Reste NR⁵ vorhanden sind, jeder Rest R⁵ gleich sein oder sich vom anderen unterscheiden kann, sowie ihrer Isomere für die Herstellung von Medikamenten zur Verwendung bei der Behandlung oder Prophylaxe menschlicher oder tierischer krebsartigen Erkrankungen, die zu einer hohen und anormalen zellulären Produktion von Desoxyribonukleinsäure führen; mit Ausnalime von viralen Erkrankungen, insbesondere Infektionen durch Viren aus der Gruppe der Retroviren.

**14.** Verwendung nach Anspruch 12, für die Herstellung von Medikamenten gegen Krebserkrankungen, wie Medikamenten gegen Leukämie und Tumore.
